# EUROPEAN PATENT APPLICATION

(11) **EP 3 031 479 A1**
(43) Date of publication of application: **15.06.2016**
(21) Application number: 14307017.5
(22) Date of filing: 12.12.2014
(51) Int. Cl.: A61L 27/18, A61L 27/22, A61L 27/24, A61L 27/46, A61L 27/56

(54) **Compositions for bone reconstruction**

(71) Applicant: Biomatlante, 44360 Vigneux de Bretagne (FR); INSERM (Institut National de la Santé et de la Recherche Médicale), 75013 Paris (FR); Université de Nantes, 44035 Nantes (FR)
(72) Inventor: DACULSI, Guy, 44360 Vigneux de Bretagne (FR); MIRAMOND, Thomas, 49100 Angers (FR); GOBIN, Chantal, 44360 Vigneux de Bretagne (FR)
(74) Representative: Cabinet Plasseraud

(57) **Abstract**

The present invention relates to a composition comprising:
a. bioactive bioceramic hollow beads comprising an opening in the bead wall, said beads having a size in the range of from 80µm to 3000µm, said opening in the bead wall having a diameter of 60µm to 2500µm, said bead wall having a thickness of 20µm to 1000µm and a microporosity of less than 10µm of at least 3%, and
b. a compound chosen from proteins and micro- or nanofibers.

## Description

The present invention relates to a composition comprising:
a. bioactive bioceramic hollow beads comprising an opening in the bead wall, said beads having a size in the range of 80µm to 3000µm, said opening in the bead wall having a diameter of 60µm to 2500µm, said bead wall having a thickness of 20µm to 1000µm and a microporosity of less than 10µm of at least 3%, used alone or combined with
b. a compound chosen from proteins and micro- or nanofibers,

The field of orthobiologics combines recent advancement in biotechnology with material sciences and tissue biology to help body's natural capacity to regenerate and repair musculoskeletal tissues. This new generation of products is expected to expand treatment options, improve patient quality of life and reduce health care costs. The repair of tissue, whether resulting from degenerative conditions or injury, is normally undertaken through surgical procedures, involving mechanical devices to help the healing process. Through scientific advances, it is widely known that human tissue, particularly bone, has a unique regenerative ability, enabling it to maintain its integrity and repair itself when fractured or damaged.

Bone grafts are supplementary bone materials used to replace existing natural bone that has been damaged by trauma or disease. These materials have a well-recognized role in orthopaedics, maxillo-facial and spine surgery. The demand has been increasing steadily during the last few years. Currently, bone autograft represents the gold standard in the vast majority of the procedures. Indeed, such a procedure has unique biological properties as it is osteoinductive, osteoconductive and completely biocompatible. These characteristics should be present in an ideal bone substitute.

However, when extensive grafting is required in patients with osteoporosis, adequate amount of good quality autologous bone may not be available. In addition, the harvesting of autologous anterior iliac crest bone graft is associated with postoperative pain and donor site morbidity. It can be associated with short- and long-term morbidity in up to 22% of cases (J.G. Seiler et al, "Iliac crest autogenous bone grafting: donor site complications", J South Orthop Assoc. 2000, 9;91-97; E.D. Arrington et al, "Complications of iliac crest bone graft harvesting" Clin Orthop 1996, 300-309; J.C. Banwart et al, "Iliac crest bone graft harvest donor site morbidiy. A statistical evaluation", Spine 1995, 20:1055-1060). The most frequently reported problems include postoperative pain, wound hematoma, infection, pelvic fracture, nerve palsy, and chronic donor site pain. The potential for donor-site morbidity has fuelled the search for non-autologous materials as alternatives. Allograft bone has been used as an alternative, but it has low osteogenicity, increased immunogenicity and resorbs more rapidly than autologous bone (F.H. Norman-Taylor et al, "Bone allograft: a cause for concern?" J Bone Joint Surg Br, 1997, 79:178-180; A.D. Aaron et al, "Allograft use in orthopedic surgery", Orthopedics 1994, 17:41-48). Moreover, transmission of disease remains a serious concern; in particular, transmission of HIV and hepatitis C has been reported. Sterilization by gamma irradiation or ethylene oxide can be used to enhance safety. These processes also adversely affect the structural integrity of the material and its osteoinductive and osteogenic activity.

Due to limited amount of autologous bone, donor site morbidity, inferior properties of allogenous bone and risk of transfer of severe diseases, there is a great global need for development of bone graft and bone substitute materials presenting high affinity for cells and tissues (like bone, cartilage, bone marrow or blood vessels), easy to handle for surgical application.

It is an object of the present invention to provide a biocompatible and biodegradable implant, which overcomes the aforementioned problems associated with materials and methods of implantation and/or insertion into bone cavities or extraction wounds. A biocompatible and biodegradable implant is to be provided which upon insertion/implantation assists in the reduction of a loss of bone volume.
It is a further object of the present invention to provide an implant which may be assembled and shaped easily in the desired manner to a defect-analogous implant in order to avoid hollow spaces between the implant and the sidewalls of the cavity.

It is a further object of the present invention to provide an implant allowing tissue ingrowth. The properties of the biocompatible implant shall be such that it also may be used for reduction of bacterial growth and infection in a bone wound and the like.
It is still a further object of the invention to provide a method for a fast and comparably simple and cost effective fabrication of the biocompatible and biodegradable implant.

Thus, the present invention proposes a composition comprising hollow beads with specific size and shaping (concavities), and which are combined to specific compounds. This composition is able to promote higher surface area for cells spreading and osteogenic cell differentiation for bone ingrowth. Such a composition is easy to prepare, and has very specific features, particularly in terms of size and shaping. It allows the living cells (Mesenchymal Stromal or Stem Cells MSCs, Osteoblasts, Induced Pluripotent Stem Cells IPS...) grow into the concavity of the hollow beads and gradually involve osteogenesis.
The composition according to the invention is preferably used as an implant which may be in different forms like pellets, hollow cylinders (tubes), parallelepiped blocs, paste, putties or membranes.

The present invention relates to a composition comprising:
a. bioactive bioceramic hollow beads comprising an opening in the bead wall, said beads having a size in the range of from 80µm to 3000µm, said opening in the bead wall having a diameter of 60µm to 2500µm, said bead wall having a thickness of 20µm to 1000µm and a microporosity of less than 10µm of at least 3%, and
b. a compound chosen from proteins and micro- or nanofibers

The composition of the present invention may be used as an implant, such as a bone or dental substitute.
The terms "implant" and "scaffold" are interchangeable in the present application, and refer to a composition according to the invention comprising components a. and b. which may be used in an animal body, preferably a human body.

The composition of the invention has osteogenic and osteoinductive properties, as shown for example in Example 2. By "osteoinductive properties" of a material, it is meant that said material can stimulate the conversion of stem cells into osteoprogenitor cell types, capable of formation of bone. By "osteoconductive properties" of a material, it is meant that said material does not have the capacity to induce bone formation. The key role of an osteoconductive material is to provide a biocompatible scaffold, which can be used by local osseous tissues to regenerate living bone. Finally, by "osteogenic properties" of a material, it is meant that said material has the inherent capacity to form bone, which implies that it comprises living cells, such as osteocytes or osteoblasts. Indeed, the hollow beads according to the invention particularly support the niche concept for local tissue microenvironments that maintain and regulate stem cells. The niche created by the hollow beads and colonized by mesenchymal stromal cells and endothelial cells is often similar to the organization of the trabecular bone. The hollow beads support the cellular complexity of the niche for bone regeneration.

The bioactive bioceramic hollow beads of the invention present a very specific structure.
Precisely, they have an optimized particle size, i.e. from 80µm to 3000µm, preferably from 80µm to 800µm.
They also possess an opening in the bead wall, said opening having a diameter of 60µm to 2500µm, preferably of 60µm to 600µm. Thus, the hollow beads show an open large internal cavity, which promotes cells spreading and osteogenic differentiation. The bead wall has a thickness of 20µm to 1000µm, preferably of 20µm to 200µm, and a microporosity of less than 10µm of at least 3%.

Preferably, the concave volumes in the hollow beads are in the range of 0.002mm³ to 14mm³, and preferably the developed open concave surfaces are in the range of 0.6mm² to 28mm².

The hollow beads of the invention are made of bioactive bioceramic. By "bioceramic", it is meant a ceramic which is biocompatible, i.e. which is inert to the body, or which is resorbable. By "bioactive", it is meant that the bioceramic has interaction with or has an effect on a cell or tissue of the body.
Preferably, said bioactive bioceramic is chosen from:
- bioactive glasses,
- apatite such as hydroxyapatite (HA, Ca₁₀(PO₄)₆(OH)₂),
- calcium phosphate such as monocalcium phosphate monohydrate (MCPM, Ca(H₂PO₄)₂.H₂O), monocalcium phosphate anhydrous (MCPA, Ca(H₂PO₄)₂), dicalcium phosphate dihydrate (DCPD, CaHPO₄.2H₂O), dicalcium phosphate anhydrous (DCP, CaPO₄), tetracalcium phosphate (TetCP, Ca₄(PO₄)₂O), calcium orthophosphate phosphate (OCP, Ca₈H₂(PO₄)₆.5H₂O), calcium pyrophosphate (CaP, Ca₂P₂O₇), α-tricalcium phosphate (α-TCP, Ca₃(PO₄)₂), β-tricalcium phosphate (β-TCP, Ca₃(PO₄)₂), biphasic calcium phosphates (BCP) and
- hydroxyapatite, α-tricalcium phosphate, β-tricalcium phosphate or biphasic calcium phosphates substituted by at least one moiety chosen from Mg, Sr, Na, Si and CO₃.
By "BCP", it is meant a mixture of hydroxyapatite with α-TCP and/or β-TCP.
Calcium phosphate bioceramics are known for their excellent biocompatibility and are therefore used in various biomedical applications, HA and TCP among them being the most used bioceramics in orthopedic and maxillo-facial applications and for the treatment of bone defects. Their close ionic similarity with the mineral components of bone, their adjustable resorption kinetics to the need of a specific therapy and their bioactive properties have been mentioned before in the prior art.

Preferably, the bioactive bioceramic is chosen from biphasic calcium phosphates consisting of hydroxyapatite and β-tricalcium phosphate. More preferably, the bioactive bioceramic is chosen from biphasic calcium phosphates having a weight ratio hydroxyapatite : β-tricalcium phosphate comprised between 10:90 and 90:10, preferably between 20:80 and 60:40, more preferably of around 20:80, 40:60 or 60:40.

The hollow beads of the composition according to the invention may be obtained by the following process:
i) mixing a porogen agent, said porogen agent being preferably chosen from naphthalene, polyethylene, camphor, poly(methyl methacrylate), polyurethane, sugar and starch, with water and bioactive bioceramic;
ii) heating the mixture of step i), to obtain sublimation or calcination of the porogen agent. At the end of this step ii), the organic phase (i.e. comprising water and the porogen agent) is volatilized or oxidized, leaving calibrated spaces behind; and
iii) sintering the mixture obtained after step ii) at a temperature of from 1000°C to 1200°C during at least 5 hours.

In step i), a mixture of water, porogen agent and the bioactive bioceramic is used. Preferably, the weight ratio water:bioactive bioceramic is comprised between 5% and 15%. A porogen agent is also present. Said porogen agent is preferably in powder form, and is preferably chosen from naphthalene, polyethylene, camphor, poly(methyl methacrylate), polyurethane, sugar and starch. It is preferably naphtalen powder. Preferably, the weight ratio porogen agent: (mixture of water and bioactive bioceramic) is comprised between 40% and 60 %.
The mixture of step i) is preferably performed using a three dimension kinematics tumbler mixer (Bioengineering Inversina Tumbler Mixer).

Then, the mixture obtained in step i) is heated, preferably in a furnace, to obtain sublimation or calcination of the porogen agent: this corresponds to step ii). The sublimation or calcination is performed at a temperature which depends on the porogen agent.
Preferably when the porogen agent is naphthalene, the sublimation or calcination is performed at around 80°C during 48 hours.
When the porogen agent is polyethylene, camphor, poly(methyl methacrylate), polyurethane, sugar or starch, the sublimation or calcination is performed at a temperature of from 200°C to 400°C during 6 to 12 hours.
After the heating step ii) (i.e. sublimation or calcination), the mixture obtained at the end of step ii) is submitted to step iii): sintering said mixture by using a higher temperature, i.e. of from 1000°C to 1200°C during at least 5 hours. Said step iii) may be performed with a high temperature furnace with gas evacuation, and controlling temperature, and finally a heating and cooling time. Preferably the temperature increase is of 0.5°C to 2°C/min during sintering, and the temperature decrease during cooling is of 0.5°C to 1°C/min.
At the end of step iii), hollow beads are obtained.
After the sintering step iii), the resulting hollow beads may be passed through sieves of a required size, so as to obtain hollow beads of a defined size. Thus, optionally, the hollow beads of the composition according to the invention may be obtained by the above defined process, further comprising a step iv) of passing the hollow beads of step iii) though sieves of a defined size.

Besides the hollow beads, the composition according to the invention also comprises a compound chosen from proteins and micro- or nanofibers.

The micro- or nanofibers are preferably obtained by an electrospinning process. Briefly, this consists in submitting a liquid to an electrical charge. Said liquid may be a polymer solution, a sol-gel, or a particulate suspension. Typically, a spinneret, like a hypodermic syringe needle, is connected to a high-voltage (5 to 50 kV) direct current power supply, a syringe pump and a grounded collector. The spinneret is loaded with the liquid, such as the polymer solution, and the liquid, such as the polymer solution, is then extruded through the spinneret, at the Taylor cone, at a constant rate by the pump.
Preferably, the electrospun nanofibers have a diameter of 200nm to 800nm.
Preferably, the electrospun microfibers have a diameter of 1µm to 20µm.
Preferably, the electrospun micro- or nanofibers are obtained by electrospinning of a polymer solution. Indeed, the polymer in solution, when submitted to an electrical charge, is extruded by forming micro- or nanofibers. Melt electrospinning process is a possible alternative to solution electrospinning. Said polymer is also called in the present application "polymer used for electrospinning".
Said polymer is preferably biocompatible, and preferably is chosen from polyesters, polysaccharides, poly(methyl methacrylate) (PMMA), glycerides and/or phosphoacylglycerols (also known as glycerophospholipids), phosphosphingolipids, phosphonoshpingolipids, phosphosaccharolipids, poloxamer/lutrol hydrogels and natural polymers.

Among the polyesters which may be used, one can select poly(α-hydroxyesters), poly(ortho esters), poly(ester amides), poly(ethylene fumarate), poly(lactic-co-glycolic) acid, poly(lactic acid) (PLA), poly(glycolic acid), poly(L-lactide-co-D,L-lactide) (PLDLLA) or polycaprolactone.
Among the polysaccharides which may be used, one can select chitin, chitosan, hyaluronic acid or cellulose derived polymers. Preferably, cellulose derived polymers are hydrogels derived from cellulose (such as cellulose ethers) in isotonic aqueous solution for injectable preparation. The cellulose derived polymers may be chosen from hydroxypropylmethylcellulose, methylcellulose, hydroxyethylcellulose and carboxymethylcellulose.
Among the natural polymers which may be used, one can select collagen of human or non human animal origin; fibrinogen like human fibrinogen; or fibrin.

Preferably, the polymer is chosen from poly(methylmethacrylate), polycaprolactone, poly(lactic-co-glycolic) acid, poly(lactic acid), poly(glycolic acid), poly(L-lactide-coD,L-lactide), chitin, chitosan, collagen, hydroxypropylmethylcellulose, methylcellulose, hydroxyethylcellulose and carboxymethylcellulose.

Besides the hollow beads, the composition according to the invention may also comprise a compound chosen from proteins. Among the proteins which may be used, one can select collagen, fibrinogen and fibrin.

The composition of the invention may be prepared by mixing the hollow beads a. with the compound b. Said mixture may then be submitted to a molding step, and finally to a drying step, so as to remove excess water.
Thus, another object of the invention is a process for preparing a composition according to the invention, comprising the mixture of the hollow beads a. with the compound b., followed by a molding step, and then a drying step.

Preferably, the mixture of the hollow beads a. with the compound b. is performed with a weight ratio hollow beads a.: component b. comprised between 10% and 60%.
The mixture of the hollow beads a. with the compound b. is preferably performed by rotative mixing, and preferably thanks to Inversina Tumbler Mixer.
Then, the resulting mixture is molded, using various moulds of shape adapted to the desired final shape. For example, pellets, parallelepipedic blocs, cylinders, hollow cylinders (tubes) or any other desired shape may be obtained. Preferably, this step is performed thanks to silicon moulds.
Alternatively, the mixture of the hollow beads a. with the compound b. is preferably performed by a simple process of multi-coating using electrospinning (ES). Said process consists in applying high voltage on a needle during the extrusion of a liquid solution containing one of the above described polymers used for electro spinning. Said polymer is biocompatible and resorbable and is of synthetic origin (like polycaprolactone (PCL), Polymethylmethacrylate (PMMA), Polylactic acid (PLA), Polyglycolic acid (PGA) and copolymers thereof such as PLGA) or of natural origin (like collagen, chitosan, alginate or hyaluronic acid). The advantage of this technology is to control the size of the nano and/or micro fibers, the density and the performance of the biomedical scaffold (implant) according to the polymer selected, as well as other various parameters. The resulting product is a non-woven fibers 3D matrix which includes the hollow beads. Said process is described to be "the sandwich process". It specifically comprises the following steps:
- Step a) consists in obtaining pellets of micro- or nanofibers by electro spinning, followed by deposition of the hollow beads (preferably without beads overlapping) thereon. Preferably, said fibers are 3D fibers, preferably of 0.1mm to 1mm in thickness, on which the hollow beads are deposited.
- Step b) consists in coating the structure obtained in a) by new pellets of micro-or nanofibers by electrospinning (3D fibers), preferably of 0.1mm to 1mm in thickness.
After steps a) and b), a 3D sandwich implant containing microfibers and the hollow beads is obtained.
For shapes different from a membrane, such as blocks or tubes, the above process can be performed by reproducing step a) and step b), followed by a step c) of coating the structure obtained in b) by new pellets of 3D fibers, preferably of 0.1mm to 1mm in thickness. This results in multicoated hollow bead implants.

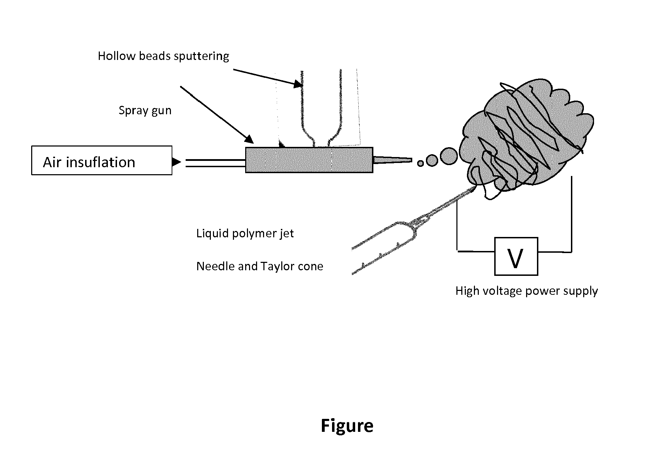

For step a), deposition of the hollow beads will be realized by mechanical deposition, or preferably by air spray method (AS), such as sputtering powder method. The implants obtained according to the sandwich process are thus obtained by the combination of Electrospinning (ES) and Air spray (AS) technologies.
The combination of ES and AS will enable multiphasic micro fibers to integrate hollow beads. The strong added-value of this combined technology lies in the control of a smart 3D scaffold with smart fiber orientations, interconnections and larger micro 3D structured scaffolds, as shown in the Figure. These two projection systems can be performed in one step, or separately one after the other.

The legend of the Figure is as follows:
Original combination of ES and AS technologies to develop new biomaterials.

The present invention will be better understood by the following examples.

### Example 1: preparation of the bioactive bioceramic hollow beads, and comparison with classical round granules

### 1. Materials & methods

### 1.1 Granule Synthesis

Different technologies were used for granules elaboration using Calcium Deficient Apatite (CDA) powders. 2 types of granules were synthesized: classical Round shape (control) and Hollow Shell shape (hollows beads according to the invention) only based on hydroxyapatite phase.
CDA powders were obtained from Biomatlante company (Vigneux-de -Bretagne, France).
Both hollow beads and round granules were synthesized from CDA powders by two different ways. The main difference is for hollow beads, where naphtalen porogen powder was used and mixed with CDA powder, under rotative mixture. Sublimation of this porogen induced the formation of an internal hollow concavity, and the resultant granule (hollow bead) morphology was shell-like.
Round granules and hollow beads were passed through sieves so as to keep respectively 400-600µm and 400-650µm granules.

### 1.2 Physico-chemical characterizations

Physico-chemical characterizations of those granules were carried-out by X-ray diffraction (XRD), Fourier transform infra-red (FTIR), Brunauer-Emmett-Teller (BET) method for specific surface area (SSA), Mercury porosimetry, and visualized by Scanning electron microscopy (SEM). Granule shape, crystal size, pores distribution and specific surface area were the main studied parameters.
All granules were previously manually crushed in agate mortar to have homogeneous particles before analyzing by XRD, FTIR.
X-ray diffraction (PW 1830 generator, Philips) was performed to check crystalline phases using Kα Cupper radiation (λ=1.5418 A) with a scan step time of 2s and step size of 0.002° (2θ).
Pellets containing 1.5% of sample were used to record Fourier transform infrared spectra (Magna IR 550, Nicolet Instruments). This method allows controlling whether undesirable chemical groups, such as carbonates and pyrophosphates (Bohic et al., 1998, Transmission FT-IR microspectroscopy of mineral phases in calcified tissues. Comptes Rendus de l'Académie des Sciences-Series III-Sciences de la Vie 321: 865-876), are avoided during synthesis process.
Samples were platinium-gold sputtered before microstructure observation using scanning electron microscopy (LEO 1450VP, Zeiss).
Specific Surface Area (SSA) measurements were determined using Brunauer-Emmett-Teller method (BET) by nitrogen adsorption at 77K (Asap2010, Micromeritics).
Pore distributions were assessed by mercury intrusion porosimetry (Autopore IV 9500, Micromeritics). Prior to analysis samples were degassed to remove physisorbed gas. Low pressure test was performed followed by high pressure test. The pressure was then varied from 0 to 30000 psi.

### 2. Results

### 2.1 SEM-EDX

Macro- and microstructures were controlled by SEM. Shape and sizes of granules were verified as homogeneous in each lot. Both macro- and microstructures have been verified as homogeneous for each type of granules. Microstructures comparison leads to conclude that round granules (control) have less microporosities than hollow beads of the invention.

Hollow bead grain boundaries enable to approximate that grain size are inferior to 1 µm in diameter. Hollow bead wall thickness was directly determined by measuring on SEM pictures; it was found to be thin, around 15µm ± 5.

### 2.2 Infra-red spectroscopy

All synthesis processes lead to controlled chemical composition, without undesirable lattice substitutions. Briefly phosphate and dihydrogen phosphate (PO4³- ,HPO4²⁻) appears respectively between 900-1100 cm⁻¹ and 550-600 cm⁻¹ while apatitic hydroxyle peak is visible at 3550 cm⁻¹ (Gadaleta et al., 1996, Fourier transform infrared spectroscopy of the solution-mediated conversion of amorphous calcium phosphate to hydroxyapatite: new correlations between X-ray diffraction and infrared data. Calcified Tissue International 58: 9-16).

### 2.3 X-Ray diffraction

Diffractograms of manually grinded granules showed that only HA phase was present as expected on both granules types. No undesirable phase was found, such as calcium oxide (CaO) or tetracalcium phosphate (TTCP) (Best et al., 2008, Bioceramics: past, present and for the future. Journal of the European Ceramic Society 28: 1319-1327; Obadia et al., 2007, Calcium-deficient apatite synthesized by ammonia hydrolysis of dicalcium phosphate dihydrate: Influence of temperature, time, and pressure. Journal of Biomedical Materials Research Part B: Applied Biomaterials 80: 32-42). Main peaks of HA appears at 2θ values of 31.75° which are correlated to preferential crystallographic axis direction [1;2;1]. Round granules and hollow beads presented equivalent diffractogram with the same full width at half maximum, which indicates a same level of high cristallinity.

### 2.4 Specific surface area (SSA) and developed surface

The specific hollow beads developed have a SSA up to 2 m²/g when round granules presented a SSA up to 1 m²/g (table 1).

**Table 1: SSA and developed surface estimation of different types of granules**

| | **SSA m²/g (n=2)** | **Developed surface mm²/granule (calculi)** |
|---|---|---|
| **Round granules 400-600µm** | **1.0 (0,1)** | **Πr² up to 0,28** |
| **Hollow beads 400-600µm** | **1.9 (0,1)** | **Πr² + Π(r-30)² up to 0,5** |

These specific surface areas are available to protein adsorption and body fluids absorption (Habibovic et al., 2005, 3D microenvironment as essential element for osteoinduction by biomaterials. Biomaterials 26: 3565-3575; Malard et al., 2007, In Vivo Demonstration of 2 Types of Microporosity on the Kinetic of Bone Ingrowth and Biphasic Calcium Phosphate Bioceramics Resorption Key Engineering Materials 361-363: 1233-1236). In order to have an approximation of surface available to cell adhesion, a simple calculus has been set up for each type of granule to obtain the developed surface: round granule has been considered as spheres whose surface directly corresponds to the surface visible to the cells. Microporosities are not included in the calculus since once the proliferation started, cells extensions goes easily over it. Hollow bead surface has been modelized as a double sphere, an external one with a radius r equal to the sphere equivalent diameter, and the internal one as (r-30µm) which represents the external radius minus the wall thickness as described above during SEM microscopy. These estimations of developed surface demonstrated that hollow beads have double surface available to cells (developed surface) and double surface available to proteins (SSA) than round granules.

### 2.5 Mercury porosimetry

Hollow beads have only one concavity which can be considered as one macropore and microporosities, while round-shaped granules have no macropore but only micropores. All these porosity profiles are well described using mercury porosimetry method (table 2 and table 3).

**Table 2: Pore distributions and total porosity comparison between hollow beads and round granules**

| **% volume of total pores** | **450 µm to 10µm** | **10µm to 1 µm** | **1µm to 100 nm** | **Total porosity %** |
|---|---|---|---|---|
| **Round granules** | **-** | 94,4 | 5.6 | 58.7 |
| **Hollow beads** | 66.3 | 25.8 | 7.9 | 81,5 |

**Table 3: Main differences in porosity between the 2 shapes of granule**

| | Micropores | Macropores |
|---|---|---|
| Round-shaped | Yes | No |
| Hollow beads | Yes | Yes (concavity) |

Each shape has its own pore distribution profile:
Round shapes provide mainly micropores in the range of 1 to 10µm, and hollow beads have a large volume of internal concavity combined to micropores ranging from 10µm to 100nm.

Thus, hollow beads according to the invention have a specific structure.

### Example 2: osteoinduction potential of the composition of the invention

### 1. Materials & methods

Hollow BCP beads of 400 to 600 µm in diameter were deposited by amount of 50mg in culture plates "Ultra-Low Adherence" (wells of 1.9 cm²) in order to promote adhesion of the cells on beads.

PLDLLA membranes alone, composite matrices of PLDLLA/HA microparticles in a proportion of 1/500 w/v of CaP and composite matrices of PLDLLA/BCP hollow beads (i.e. composition of the invention) each containing 5mg of hollow beads, were cut to cover the surface of the same culture wells as described above.

On all these 3D matrices were cultured human mesenchymal stem cells (MSC) to a concentration of 10⁴ cells/cm², in the presence either of standard medium (CT) (α-MEM, 10% FBS, 1% L-glutamine, 1 % Penicillin / Streptomycin) or osteogenic medium (OST) (standard medium supplemented with 50 µM ascorbic acid, 0.1 µM dexamethasone, 100mM β-glycerophosphate).

Control is represented by 2D cultures under the same conditions (CT and OST). After 7, 14, 21 and 28 days of culture, the RNA was extracted by the Trizol method and treated with DNase to eliminate genomic DNA.

The RNA was quantified by Nanodrop (ND-1000, Labtech), the ratio of signals acquired at 260 nm / 280 nm indicating the integrity of RNA and the ratio of signals acquired at 260 nm / 230 nm resulting from the purity of RNA. Ratios between 1.8 and 2.1 are considered as corresponding to those of pure RNA which could be exploited by RT-PCR. The assessment of RNA integrity was also verified on RNA chip (Agilent Bioanalyzer 2100), the RNA are considered as correct if they include a RIN (RNA Integrity Number) greater than or equal to 7 on a scale of 10. Real time PCR was carried out after a step of reverse transcription of RNA (to obtain cDNA) by the method of SYBR Green (MX3000P, Stratagen). Two reference genes (ACTB and PP1A) helped to normalize the results.

### 2.Results

Day 28 (D28) provides results regarding the influence of matrices (3D vs 2D) and the induction of differentiation in osteogenic medium (OST). These results are shown in table 4 below.

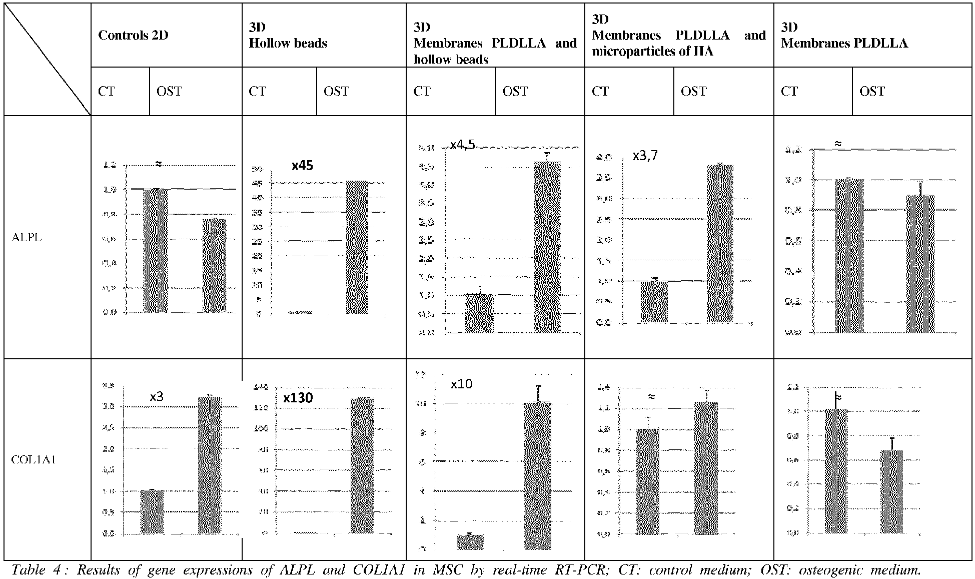

As results, only 3D matrices comprising CaP present at D28 a significant increase of gene expression of ALPL and COL1A1.
The composition according to the invention allows significant increases of ALPL and COL1A1 gene expressions.

### Example 3: preparation and evaluation of compositions of the invention using a molding process

### 1. Mineralized implant with fibrin

### Process:

The composition of the invention associates fibrin and bioceramic hollow beads obtained with BCP.

Fibrin is obtained by reticulation of fibrinogen glue, such as Tissucol, with thrombin. The only requirement must be for the thrombin concentration (low concentration < 50U) to prevent rapid reticulation of the fibrinogen to fibrin 3D architecture. The fibrinogen is reticulated with thrombin diluted at 4U.
The ideal mixture was 1 volume of BCP hollow beads and 2 volumes of fibrinogen.
Mixture of BCP hollow beads, fibrinogen and thrombin 4U is molded in pellet or dedicated shaped to extract excess water, then dried in air or by a freeze drying method, so as to obtain the desired shape, such as a membrane, a strip, wedges, parralelepiped shape or cylinders.
Sandwich structure by different BCP/Fibrin ratio (0/100 to 50/50) can be realized for specific application in bone regeneration and surrounding soft tissue reconstruction. The resulting product is a resorbable elastic implant.

### Properties:

The obtained composition (implant) is hemostatic, highly permeable to biological fluid and cells colonization.
It may also be used as scaffold for tissue engineering (like bone marrow, concentrated bone marrow or MSCs).

It presents a time of resorption of less than 3 weeks, and extracellular matrix is formed at the expense of the fibrin network. The implant also presents a high elasticity (15 to 30% in elongation).

### 2. Mineralized membrane, strip, or blocks with collagen

### Process:

The composition of the invention associates collagen and bioceramic hollow beads obtained with BCP.
The collagen is not reticulated during the mixture.
The ideal mixture was 1 volume of BCP hollow beads and 2 volumes of collagen. Mixture of BCP hollow beads and collagen is molded in pellet or dedicated shaped to extract excess water, then dried in air or by a freeze drying method, so as to obtain an implant.
Sandwich structure by different BCP/collagen ratio (0/100 to 50/50) can be realized for specific application in bone regeneration and surrounding soft tissue reconstruction. Finally, a sterilization step by irradiation completes the process, involving physical reticulation of the collagen.
The resulting product is a resorbable implant with the desired shape, such as a membrane, strip, blocks or plugs.

### Properties:

The obtained composition is permeable to biological fluid and cells colonization.
It may also be used as scaffold for tissue engineering (like bone marrow, concentrated bone marrow or MSCs).
It presents a time of resorption of 6 to 24 weeks for collagen, and of 6 to 54 weeks for the hollow beads, and extracellular matrix is formed at the expense of the collagen network.

### Example 4: in vivo studies with hollow beads a. of the invention

A) Beads of classical Round shape (control, dense granules) or Hollow Shell shape (hollow beads a. according to the invention), both made of calcium phosphate, were compared in a rat model of critical size defect in femoral epiphysis.
   Rat femoral epiphysis was filled with the hollow beads or dense granules. After 4 weeks, rats were sacrificed and histological sections were made.
   Histomorphological results clearly demonstrate that bone tissue was induced inside and outside of the hollow granules with penetration of body fluids in its thin walls. More bone volume was obtained with the hollow beads than with dense granules.
   Bone marrow was encapsulated inside hollow granules concavities while dense granules permit only osteoconduction on its convex surface.
B) Beads of Hollow Shell shape (hollows beads a. according to the invention), alone or loaded with osteogenic peptide P8, were also compared in a rat model of critical size defect in femoral epiphysis. Empty defect acted as control.
   In comparison with empty defect, hollow granules of the invention, with and without loading with peptide P8, were biocompatible and highly promote bone formation (osteoconduction and osteoinduction). Control group remain without bone regeneration.

## Claims

1. Composition comprising:
a. bioactive bioceramic hollow beads comprising an opening in the bead wall, said beads having a size in the range of from 80µm to 3000µm, said opening in the bead wall having a diameter of 60µm to 2500µm, said bead wall having a thickness of 20µm to 1000µm and a microporosity of less than 10µm of at least 3%, and
b. a compound chosen from proteins and micro- or nanofibers.

2. Composition according to claim 1, wherein the bioactive bioceramic is chosen from:
- bioactive glasses,
- apatite such as hydroxyapatite,
- calcium phosphate such as monocalcium phosphate monohydrate, monocalcium phosphate anhydrous, dicalcium phosphate dihydrate, dicalcium phosphate anhydrous, tetracalcium phosphate, calcium orthophosphate phosphate, calcium pyrophosphate, α-tricalcium phosphate, β-tricalcium phosphate, biphasic calcium phosphates, and
- hydroxyapatite, α-tricalcium phosphate, β-tricalcium phosphate or biphasic calcium phosphates substituted by at least one moiety chosen from Mg, Sr, Na, Si and CO₃.

3. Composition according to claim 1 or 2, wherein the bioactive bioceramic is chosen from biphasic calcium phosphates having a weight ratio hydroxyapatite : β-tricalcium phosphate comprised between 10:90 and 90:10, preferably between 20:80 and 60:40, more preferably of around 20:80, 40:60 or 60:40.

4. Composition according to any one of claims 1 to 3, wherein the hollow beads have a size in the range of from 80µm to 800µm, and the opening in the bead wall has a diameter of 60µm to 600µm.

5. Composition according to any one of claims 1 to 4, wherein the concave volumes in the hollow beads are in the range of 0.002mm³ to 14mm³, and preferably the developed open concave surfaces are in the range of 0.6mm² to 28mm².

6. Composition according to any one of claims 1 to 5, wherein the bead wall has a thickness of 20µm to 200µm.

7. Composition according to any one of claims 1 to 6, wherein the electrospun micro- or nanofibers are obtained by electrospinning of a polymer solution.

8. Composition according to claim 7, wherein the polymer is biocompatible, preferably chosen from polyesters, polysaccharides, poly(methyl methacrylate), glycerides and/or phosphoacylglycerols (also known as glycerophospholipids), phosphosphingolipids, phosphonosphingolipids, phosphosaccharolipids, poloxamer/lutrol hydrogels and natural polymers.

9. Composition according to claim 7 or 8, wherein the polymer is chosen from poly(methylmethacrylate), polycaprolactone, poly(lactic-co-glycolic) acid, poly(lactic acid), poly(glycolic acid), poly(L-lactide-co-D,L-lactide), chitin, chitosan, collagen, hydroxypropylmethylcellulose, methylcellulose, hydroxyethylcellulose and carboxymethylcellulose.

10. Composition according to any one of claims 1 to 7, wherein the protein is chosen from collagen, fibrinogen and fibrin.

11. Composition according to any one of claims 1 to 10, for use as an implant such as a bone or dental substitute.

12. Composition according to any one of claims 1 to 10, wherein the hollow beads are obtained by:
i) mixing a porogen agent with water and bioactive bioceramic, said porogen agent being preferably chosen from naphthalene, polyethylene, camphor, poly(methyl methacrylate), polyurethane, sugar and starch;
ii) heating the mixture of step i), to obtain sublimation or calcination of the porogen agent; and
iii) sintering the mixture obtained after step ii) at a temperature of from 1000°C to 1200°C during at least 5 hours.

13. Process for preparing a composition according to any one of claims 1 to 10 or 12, comprising the mixture of the hollow beads a. with the compound b., followed by a molding step, and then a drying step.

14. Process for preparing a composition according to any one of claims 1 to 10 or 12, comprising the following steps:
a) obtaining pellets of micro- or nanofibers by electro spinning, followed by deposition of the hollow beads thereon, preferably by air spray method, and
b) coating the structure obtained in a) by new pellets of micro- or nanofibers by electrospinning.

15. Process according to claim 14 which comprises a step c) of coating the structure obtained in b) by new pellets of micro- or nanofibers by electrospinning.
